# EUROPEAN PATENT APPLICATION

(11) **EP 1 859 677 A1**
(43) Date of publication of application: **28.11.2007**
(21) Application number: 06715244.7
(22) Date of filing: 27.02.2006
(51) Int. Cl.: A01K 67/027, C12N 15/09, G01N 33/15, G01N 33/50

(54) **DIABETES MODEL ANIMAL**

(30) Priority: 25.02.2005 JP 2005051692
(71) Applicant: Tokyo Metropolitan Organization for Medical Research, Shinjuku-ku, Tokyo 163-8001 (JP); National University Corporation Nara Institute of Science and Technology, Ikoma-shi, Nara 630-0192 (JP)
(72) Inventor: YONEKAWA, Hiromichi, ama, 3370007 (JP); MATSUOKA, Kunie, Tokyo 1560055 (JP); SHITARA, Hiroshi, Chiba, 2710044 (JP); KOHNO, Kenji, c/o National University Corporation Nara, Ikoma-shi, Nara 6300192 (JP)
(74) Representative: Woods, Geoffrey Corlett
(86) International application number: PCT/JP2006/304185
(87) International publication number: WO 2006/090918

(57) **Abstract**

The present invention relates to a diabetes animal model. Specifically, the present invention relates to a transgenic nonhuman animal, into which recombinant DNA comprising a gene encoding a diphtheria toxin receptor and an insulin promoter for regulating expression of the above gene has been introduced.

SEQUENCE LISTING

<110> TOKYO METROPOLITAN ORGANIZATION FOR MEDICAL RESEARCH
NATIONAL UNIVERSITY CORPORATION NARA INSTITUTE OF SCIENCE AND
TECHNOLOGY

<120> DIABETES MODEL ANIMAL

<130> G07-0081

<140> PCT/JP2006/304185
<141> 2006-02-27

<150> JP2005-051692
<151> 2005-02-25

<160> 5

<170> PatentIn version 3.3

<210> 1
<211> 2360
<212> DNA
<213> Homo sapiens

<400> 1
   gctacgcggg ccacgctgct ggctggcctg acctaggcgc gcggggtcgg gcggccgcgc 60
   gggcgggctg agtgagcaag acaagacact caagaagagc gagctgcgcc tgggtcccgg 120
   ccaggcttgc acgcagaggc gggcggcaga cggtgcccgg cggaatctcc tgagctccgc 180
   cgcccagctc tggtgccagc gcccagtggc cgccgcttcg aaagtgactg gtgcctcgcc 240
   gcctcctctc ggtgcgggac catgaagctg ctgccgtcgg tggtgctgaa gctctttctg 300
   gctgcagttc tctcggcact ggtgactggc gagagcctgg agcggcttcg gagagggcta 360
   gctgctggaa ccagcaaccc ggaccctccc actgtatcca cggaccagct gctaccccta 420
   ggaggcggcc gggaccggaa agtccgtgac ttgcaagagg cagatctgga ccttttgaga 480
   gtcactttat cctccaagcc acaagcactg gccacaccaa acaaggagga gcacgggaaa 540
   agaaagaaga aaggcaaggg gctagggaag aagagggacc catgtcttcg gaaatacaag 600
   gacttctgca tccatggaga atgcaaatat gtgaaggagc tccgggctcc ctcctgcatc 660
   tgccacccgg gttaccatgg agagaggtgt catgggctga gcctcccagt ggaaaatcgc 720
   ttatatacct atgaccacac aaccatcctg gccgtggtgg ctgtggtgct gtcatctgtc 780
   tgtctgctgg tcatcgtggg gcttctcatg tttaggtacc ataggagagg aggttatgat 840
   gtggaaaatg aagagaaagt gaagttgggc atgactaatt cccactgaga gagacttgtg 900
   ctcaaggaat cggctgggga ctgctacctc tgagaagaca caaggtgatt tcagactgca 960
   gaggggaaag acttccatct agtcacaaag actccttcgt ccccagttgc cgtctaggat 1020
   tgggcctccc ataattgctt tgccaaaata ccagagcctt caagtgccaa acagagtatg 1080
   tccgatggta tctgggtaag aagaaagcaa aagcaaggga ccttcatgcc cttctgattc 1140
   ccctccacca aaccccactt cccctcataa gtttgtttaa acacttatct tctggattag 1200
   aatgccggtt aaattccata tgctccagga tctttgactg aaaaaaaaaa agaagaagaa 1260
   gaaggagagc aagaaggaaa gatttgtgaa ctggaagaaa gcaacaaaga ttgagaagcc 1320
   atgtactcaa gtaccaccaa gggatctgcc attgggaccc tccagtgctg gatttgatga 1380
   gttaactgtg aaataccaca agcctgagaa ctgaattttg ggacttctac ccagatggaa 1440
   aaataacaac tatttttgtt gttgttgttt gtaaatgcct cttaaattat atatttattt 1500
   tattctatgt atgttaattt atttagtttt taacaatcta acaataatat ttcaagtgcc 1560
   tagactgtta ctttggcaat ttcctggccc tccactcctc atccccacaa tctggcttag 1620
   tgccacccac ctttgccaca aagctaggat ggttctgtga cccatctgta gtaatttatt 1680
   gtctgtctac atttctgcag atcttccgtg gtcagagtgc cactgcggga gctctgtatg 1740
   gtcaggatgt aggggttaac ttggtcagag ccactctatg agttggactt cagtcttgcc 1800
   taggcgattt tgtctaccat ttgtgttttg aaagcccaag gtgctgatgt caaagtgtaa 1860
   cagatatcag tgtctccccg tgtcctctcc ctgccaagtc tcagaagagg ttgggcttcc 1920
   atgcctgtag ctttcctggt ccctcacccc catggcccca ggccacagcg tgggaactca 1980
   ctttcccttg tgtcaagaca tttctctaac tcctgccatt cttctggtgc tactccatgc 2040
   aggggtcagt gcagcagagg acagtctgga gaaggtatta gcaaagcaaa aggctgagaa 2100
   ggaacaggga acattggagc tgactgttct tggtaactga ttacctgcca attgctaccg 2160
   agaaggttgg aggtggggaa ggctttgtat aatcccaccc acctcaccaa aacgatgaag 2220
   gtatgctgtc atggtccttt ctggaagttt ctggtgccat ttctgaactg ttacaacttg 2280
   tatttccaaa cctggttcat atttatactt tgcaatccaa ataaagataa cccttattcc 2340
   ataaaaaaaa aaaaaaaaaa 2360

<210> 2
<211> 1895
<212> DNA
<213> Homo sapiens

<400> 2
gcatgccaat tcgagctcgc ccgatcctgg atctcagctc cctggccgac aacactggca 60

aactcctact catccacgaa ggccctcctg ggcatggtgg tccttcccag cctggcagtc 120

tgttcctcac acaccttgtt agtgcccagc ccctgaggtt gcagctgggg gtgtctctga 180

agggctgtga gcccccagga agccctgggg aagtgcctgc cttgcctccc cccggccctg 240

ccagcgcctg gctctgccct cctacctggg ctccccccat ccagcctccc tccctacaca 300

ctcctctcaa ggaggcaccc atgtcctctc cagctgccgg gcctcagagc actgtggcgt 360

cctggggcag ccaccgcatg tcctgctgtg gcatggctca gggtggaaag ggcggaaggg 420

aggggtcctg cagatagctg gtgcccacta ccaaacccgc tcggggcagg agagccaaag 480

gctgggtgtg tgcagagcgg ccccgagagg ttccgaggct gaggccaggg tgggacatag 540

ggatgcgagg ggccggggca caggatactc caacctgcct gcccccatgg tctcatcctc 600

ctgcttctgg acctcctgat cctgcccctg gtgctaagag gcaggtaggg gctgcaggca 660

gcagggctcg gagcccatgc cccctcacca tgggtcaggc tggacctcca ggtgcctgtt 720

ctggggagct gggagggccg gaggggtgta ccccaggggc tcagcccaga tgacactatg 780

ggggtgatgg tgtcatggga cctggccagg agaggggaga tgggctccca gaagaggagt 840

gggggctgag agggtgcctg gggggccagg acggagctgg gccagtgcac agcttcccac 900

acctgcccac ccccagagtc ctgccgccac ccccagatca cacggaagat gaggtccgag 960

tggcctgctg aggacttgct gcttgtcccc aggtccccag gtcatgccct ccttctgcca 1020

ccctggggag ctgagggcct cagctggggc tgctgtccta aggcagggtg ggaactaggc 1080

agccagcagg gaggggaccc ctccctcact cccactctcc cacccccacc accttggccc 1140

atccatggcg gcatcttggg ccatccggga ctggggacag gggtcctggg gacaggggtc 1200

tgaggacagg ggtgtgggca caggggtcct ggggacaggg gtcctgggga caggggtcct 1260

ggggacaggg gtctggggac agcagcgcaa agagccccgc cctgcagcct ccagctctcc 1320

tggtctaatg tggaaagtgg cccaggtgag ggctttgctc tcctggagac atttgccccc 1380

agctgtgagc agggacaggt ctggccaccg ggcccctggt taagactcta atgacccgct 1440

ggtcctgagg aagaggtgct gacgaccaag gagatcttcc cacagaccca gcaccaggga 1500

aatggtccgg aaattgcagc ctcagccccc agccatctgc cgaccccccc accccaggcc 1560

ctaatgggcc aggcggcagg ggttgacagg taggggagat gggctctgag actataaagc 1620

cagcgggggc ccagcagccc tcagccctcc aggacaggct gcatcagaag aggccatcaa 1680

gcaggtctgt tccaagggcc tttgcgtcag gtgggctcag ggttccaggg tggctggacc 1740

ccaggcccca gctctgcagc agggaggacg tggctgggct cgtgaagcat gtgggggtga 1800

gcccaggggc cccaaggcag ggcacctggc cttcagcctg cctcagccct gcctgtctcc 1860

cagatcactg tccttctgca ggaagcaggg atcgg 1895

<210> 3
<211> 627
<212> DNA
<213> Artificial

<220>

<223>  mutant

<220>
<221>  mutation
<222>  (442)..(447)

<400>  3
atgaagctgc tgccgtcggt ggtgctgaag ctctttctgg ctgcagttct ctcggcactg      60

gtgactggcg agagcctgga gcggcttcgg agagggctag ctgctggaac cagcaacccg     120

gaccctccca ctgtatccac ggaccagctg ctacccctag gaggcggccg ggaccggaaa     180

gtccgtgact tgcaagaggc agatctggac cttttgagag tcactttatc ctccaagcca     240

caagcactgg ccacaccaaa caaggaggag cacgggaaaa gaaagaagaa aggcaagggg     300

ctagggaaga agagggaccc atgtcttcgg aaatacaagg acttctgcat ccatggagaa     360

tgcaaatatg tgaaggagct ccgggctccc tcctgcatct gccacccggg ttaccatgga     420

gagaggtgtc atgggctgag cagcaccgtg gaaaatcgct tatataccta tgaccacaca     480

accatcctgg ccgtggtggc tgtggtgctg tcatctgtct gtctgctggt catcgtgggg     540

cttctcatgt ttaggtacca taggagagga ggttatgatg tggaaaatga agagaaagtg     600

aagttgggca tgactaattc ccactga                                         627

<210>  4
<211>  22
<212>  DNA
<213>  Artificial

<220>
<223>  primer

<400>  4
caactacatc ctggtcatca tc                                               22

<210>  5
<211>  22
<212>  DNA

<213>  Artificial

<220>
<223>  primer

<400>  5
cagacagatg acagcaccac ag                                                22

## Description

### TECHNICAL FIELD

The present invention relates to a transgenic nonhuman animal, into which recombinant DNA comprising a gene encoding a diphtheria toxin receptor and a promoter for regulating expression of the above gene has been introduced, and a diabetes animal model.

### BACKGROUND ART

The present inventors have previously developed a method known as TRECK method (International Publication WO98/33899; Saito M, et. al., (2001) Nat. Biotechnology 19: 746-750). That is to say, this method has been developed as a result of focusing attention on the fact that heparin-binding EGF is a main body of a receptor for toxin (diphtheria toxin) generated by *Corynebacterium diphtheriae.* This method uses mouse cells having resistance to diphtheria toxin added from outside of the cells, which is 1,000 times or more higher than the same type of resistance of human cells, so as to specifically disrupt only a mouse specific cell population. A summary of the TRECK method is as follows. First, a mouse cell- or tissue-specific promoter is allowed to bind to a diphtheria toxin receptor (DTR) that is specific for a human, so as to produce recombinant DNA. The produced recombinant DNA is then introduced into a mouse, so as to produce a transgenic (Tg) mouse. Because of the introduced promoter, the thus produced Tg mouse is able to express DTR specifically on the surface of a cell. Thus, if diphtheria toxin (DT) is administered to this Tg mouse, it becomes possible to eliminate only targeted cells in any given period, or to give transient damage to the mouse. To date, this method has already been used to create a human disease model such as a model of hepatitis. Application of this method would enable creation of various types of models.

By the way, diabetes is a lifestyle-related disease. It is said that the number of individuals affected by diabetes is estimated to be 16,000,000. Diabetes involves a state in which the level of glucose in blood excessively increases (hyperglycemia) due to insufficient insulin action, namely, shortage of insulin supply, and a decrease in sensitivity in an insulin target organ, and as a result, glucose in blood floods into urine (glucosuria). In reality, such a state is diagnosed as diabetes by measuring the level of glucose contained in blood (blood glucose level). In addition, it has been known that diabetes causes complications such as nervous disorder, retinopathy, or nephropathy. Moreover, it is said that a metabolic syndrome that is the combination of diabetes with other lifestyle-related diseases such as adiposis, hyperlipidemia, and hypertension, becomes a main cause of cardiovascular diseases such as myocardial infarction or cerebral infarction. Accordingly, investigation to determine the cause of diabetes and the establishment of a radical preventive or therapeutic method based on such investigation have been considered to be extremely important, and thus production of a diabetes animal model has been desired. In particular, for the radical cure of type I diabetes, transplantation of the stem cells or precursor cells of pancreatic β cells obtained using regenerative medicine has obtained a high degree of expectation. A suitable animal model of type I diabetes that uses regenerative medicine should satisfy the following three conditions:
1) the animal model certainly develops diabetes as a result of administration of an agent only once, in spite of difference in the animal's genetic background, species difference, or gender difference;
2) the administered agent is rapidly eliminated from the body of the animal model in a short time after the onset of diabetes, and thus the agent does not affect the animal model other than the onset of diabetes; and
3) it is possible to transplant the cells of almost all mammals including a human as a typical example to the animal model.

However, a suitable animal model that satisfies the aforementioned conditions has not yet been established.

### DISCLOSURE OF THE INVENTION

The present invention provides a transgenic nonhuman animal, into which recombinant DNA comprising a gene encoding a diphtheria toxin receptor and a promoter for regulating expression of the above gene has been introduced.

The present inventor has conducted intensive studies directed towards achieving the aforementioned object. The inventor has applied the TRECK method to produce a transgenic animal, into which recombinant DNA comprising an insulin promoter has been introduced. As a result, the inventor has found that the thus produced animal develops diabetes, thereby completing the present invention.

That is to say, the present invention is as follows.
(1) A transgenic nonhuman animal, into which recombinant DNA comprising a gene encoding a diphtheria toxin receptor and an insulin promoter for regulating expression of the above-described gene has been introduced.
   The transgenic nonhuman animal of the present invention is characterized in that it develops diabetes as a result of administration of diphtheria toxin. In addition, the gene encoding a diphtheria toxin receptor is a heparin-binding EGF gene derived from Primates, and is preferably a human heparin-binding EGF gene. The animal is any one selected from the group consisting of a mouse, a rat, a guinea pig, a hamster, a dog, a cat, a goat, a sheep, a swine, a bovine, and a horse. The animal is preferably a mouse. Moreover, the animal used in the present invention is preferably a severe combined immunodeficiency animal.
(2) A method of producing a transgenic nonhuman animal, which is characterized in that it comprises introduction of recombinant DNA comprising a gene encoding a diphtheria toxin receptor and an insulin promoter for regulating expression of the above-described gene into a nonhuman animal.
(3) A method of producing a diabetes animal model, which is characterized in that it comprises administration of diphtheria toxin to the transgenic nonhuman animal according to (1) above or a transgenic nonhuman animal produced by the method according to (2) above.
(4) A method of causing the transgenic nonhuman animal according to (1) above or a transgenic nonhuman animal produced by the method according to (2) above to develop diabetes, which comprises administration of diphtheria toxin to the above-described transgenic nonhuman animal.
(5) A diabetes animal model, which is produced by the method according to (3) above.
(6) A method of screening a therapeutic agent used for diabetes, which is characterized in that it comprises administration of a candidate substance to the diabetes animal model according to (3) above.
   In the screening method of the present invention, for example, when the blood glucose level of a diabetes animal model, with which a candidate substance has been allowed to come into contact, is measured, and when the thus measured blood glucose level is lower than the blood glucose level of a control diabetes animal model, with which such a candidate substance has not been allowed to come into contact, the above-described candidate substance can be selected as a therapeutic agent used for diabetes.
   Moreover, in the screening method of the present invention, when the insulin level (e.g. blood insulin level) of a diabetes animal model, with which a candidate substance has been allowed to come into contact, is measured, and when the thus measured insulin level is higher than the insulin level of a control diabetes animal model, with which such a candidate substance has not been allowed to come into contact, the above-described candidate substance can be selected as a therapeutic agent used for diabetes.
(7) A method of producing a transgenic nonhuman animal having pancreatic cells derived from another animal, which is characterized in that it comprises transplantation of stem cells derived from another animal to the animal according to (5) above.
   Examples of the stem cells used in transplantation include hematopoietic stem cells. Such hematopoietic stem cells are preferably bone marrow-derived cells or cord blood-derived cells.
(8) A transgenic nonhuman animal having pancreatic cells derived from another animal, which is produced by the method according to (7) above.

The pancreatic cells of this nonhuman animal include cells derived from the transplanted hematopoietic stem cells of another animal. Preferred examples of such hematopoietic stem cells include bone marrow cells and cord blood cells.

### BRIEF DESCRIPTION OF THE DRAWINGS

Figure 1 is a schematic view of a method of causing a mouse to develop diabetes according to the TRECK method.
Figure 2 shows a portion of recombinant DNA used in production of the transgenic mouse of the present invention.
Figure 3 is a photograph showing detection of the introduced gene by PCR method.
Figure 4A is a graph showing the results obtained by administering diphtheria toxin to the transgenic mouse of the present invention and then measuring the blood glucose level.
Figure 4B is a graph showing elevation of blood glucose level by administration of diphtheria toxin.
Figure 5A is a graph obtained by administering insulin to the transgenic mouse of the present invention, the blood glucose level of which has been elevated by administration of diphtheria toxin, and then by measuring the blood glucose level.
Figure 5B is a graph showing the blood insulin level of an SCID-Ins-TRECK-Tg mouse.
Figure 5C is a graph showing that the hyperglycemia of the SCID-Ins-TRECK-Tg mouse is insulin dependent.
Figure 6 includes photographs in which the pancreases of the transgenic mice of the present invention are histologically analyzed.
Figure 7 includes photographs regarding the immunohistological staining of pancreatic tissues with anti-hHB-EGF antibodies.
Figure 8 includes photographs regarding the immunohistological staining of the pancreatic tissues of the SCID-Ins-TRECK-Tg mice.
Figure 9 is a view showing that hyperglycemia is improved by transplantation of the bone marrow cells of a C57BL/6 mouse.
Figure 10 is a view showing changes in the blood glucose levels obtained after transplantation of human cord blood-derived cells.

### BEST MODE FOR CARRYING OUT THE INVENTION

The best mode for carrying out the present invention will be described below. The following embodiments are provided only for the purpose of illustrating the present invention, and thus the present invention is not limited to such embodiments. The present invention can be carried out in various embodiments unless it departs from the gist thereof.

All publications, published patent applications, patent gazette and other patent documents cited in the present specification are incorporated herein by reference in their entirety. In addition, the present specification includes part or all of the contents as disclosed in the specification, claims and/or drawings of Japanese Patent Application No. 2005-51692, which is a priority document of the present application.

The present invention relates to a transgenic nonhuman animal, into which recombinant DNA comprising a gene encoding a diphtheria toxin receptor and a promoter for regulating expression of the above gene has been introduced, and a diabetes animal model, which develops diabetes.

### 1. Recombinant DNA

The present invention is characterized in that it has acquired a transgenic animal, which develops diabetes as a result of introduction of recombinant DNA comprising a gene encoding a diphtheria toxin receptor and a promoter for regulating expression of the above gene into an animal. The recombinant DNA of the present invention will be described below.

### (1) Gene encoding diphtheria toxin receptor

The main body of the diphtheria toxin receptor of the present invention is heparin-binding EGF (HB-EGF; heparin binding-epidermal growth factor) derived from Primates. The above heparin-binding EGF gene includes a membrane-bound type and a free type obtained by action of protease. In the present invention, a membrane-bound type is used. The nucleotide sequence of a heparin-binding EGF gene that can be used in the present invention has been known, and it can be easily obtained from public database such as GenBank. As an example, the nucleotide sequence of a human heparin-binding EGF gene is as shown in SEQ ID NO: 1 (NM_001945). However, persons skilled in the art could select and use a partial fragment of the aforementioned nucleotide sequence, such as a coding region (the region ranging from positions 262 to 888 of the nucleotide sequence as shown in SEQ ID NO: 1).

In addition, the heparin-binding EGF gene of the present invention includes not only DNA having the nucleotide sequence as shown in SEQ ID NO: 1 or a partial sequence thereof, but also DNA, which hybridizes with a sequence complementary to the nucleotide sequence as shown in SEQ ID NO: 1 or a partial sequence thereof under stringent conditions, and which encodes a region having heparin-binding EGF activity.

The term "heparin-binding EGF activity" is used herein to mean activity necessary for functioning as a membrane-bound type diphtheria toxin receptor, as stated above.

Such a heparin-binding EGF gene can be obtained from a human cDNA library and genomic library according to a known hybridization method such as colony hybridization, plaque hybridization, or Southern blotting, using DNA having the nucleotide sequence as shown in SEQ ID NO: 1 or a fragment thereof as a probe. For the details of such methods, please refer to Molecular Cloning, A Laboratory Manual 2nd ed. (Cold Spring Harbor Laboratory Press (1989)).

Examples of stringent conditions applied in the aforementioned hybridization include conditions consisting of 1 x SSC to 2 x SSC, 0.1% to 0.5% SDS, and a temperature between 42°C and 68°C. More specifically, pre-hybridization is carried out at a temperature between 60°C and 68°C for 30 minutes or longer, and hybridization is then carried out with a probe. Thereafter, the membrane is washed 4 to 6 times in 2 x SSC and 0.1% SDS at room temperature for 5 to 15 minutes. Moreover, the nucleotide sequence of a gene having homology of 50% or more, 60% or more, 70% or more, 80% or more, 90% or more, 95% or more, 98% or more, or 99% or more, with the nucleotide sequence as shown in SEQ ID NO: 1 or a partial sequence thereof (e.g. the sequence of a coding region), and encoding a protein having heparin-binding EGF activity as a diphtheria toxin receptor, can also be used.

### (2) Insulin promoter

The insulin promoter used in the present invention is a DNA element necessary for controlling specific expression in islets of Langerhans β cells of pancrea. The nucleotide sequence of the above promoter gene is publicly known. The above sequence can be easily obtained from public database such as GenBank. As an example of such an insulin promoter that can be used in the present invention, the nucleotide sequence (1.9 kb) of a human insulin promoter described in J. Exp. Med. (1998) 188(8): 1445-1451 is as shown in SEQ ID NO: 2.

In addition, the insulin promoter of the present invention includes not only DNA having the nucleotide sequence as shown in SEQ ID NO: 2, but also DNA, which hybridizes with a sequence complementary to the nucleotide sequence as shown in SEQ ID NO: 2 under stringent conditions, and which encodes a region having insulin promoter activity. The term "insulin promoter activity" is used herein to mean activity capable of promoting expression of a gene that is ligated downstream of the promoter.

Such an insulin promoter can be obtained from a human cDNA library and genomic library according to a known hybridization method such as colony hybridization, plaque hybridization, or Southern blotting, using DNA having the nucleotide sequence as shown in SEQ ID NO: 2 or a fragment thereof as a probe. For the details of such methods, please refer to Molecular Cloning, A Laboratory Manual 2nd ed. (Cold Spring Harbor Laboratory Press (1989)).

Examples of stringent conditions applied in the aforementioned hybridization include conditions consisting of 1 x SSC to 2 x SSC, 0.1% to 0.5% SDS, and a temperature between 42°C and 68°C. More specifically, pre-hybridization is carried out at a temperature between 60°C and 68°C for 30 minutes or longer, and hybridization is then carried out with a probe. Thereafter, the reaction product is washed 4 to 6 times in 2 x SSC and 0.1% SDS at room temperature for 5 to 15 minutes. Moreover, a nucleotide sequence of nucleotides having homology of 50% or more, 60% or more, 70% or more, 80% or more, 90% or more, 95% or more, 98% or more, or 99% or more, with the nucleotide sequence as shown in SEQ ID NO: 2, and having the aforementioned insulin promoter activity, can also be used.

### (3) Regulation of gene expression

The recombinant DNA of the present invention is obtained by fusing an insulin promoter that is a cell- or tissue-specific promoter to a heparin-binding EGF gene that is a diphtheria toxin receptor specific for Primates. Because of the presence of such an insulin promoter, the heparin-binding EGF gene is specifically expressed on the surface of a cell. If diphtheria toxin (DT) is administered to such an individual animal into which such recombinant DNA has been introduced, a cell for expressing an insulin promoter can be specifically disrupted at any given time. A method of producing a cloning vector comprising an expression cassette, in which an insulin promoter has been allowed to bind to a heparin-binding EGF gene, will be described below.

The heparin-binding EGF gene of the present invention that encodes a diphtheria toxin receptor and the insulin promoter of the present invention can be produced by a common chemical synthesis method or a biochemical synthesis method. For example, a nucleic acid synthesis method using a DNA synthesizer, which is commonly used as a genetic engineering method, can be used. In addition, after isolation or synthesis of a nucleotide sequence used as a template, the PCR method or a gene amplification method using a cloning vector can be applied. The sequence of the above gene may be confirmed by a known method such as TA cloning, as necessary. The aforementioned method can be easily carried out by persons skilled in the art according to the descriptions of Molecular Cloning, 2nd ed., Cold Spring Harbor Laboratory Press (1989)), etc. The obtained PCR product can be purified using GENECLEAN (Funakoshi), QIAGEN (QIAGEN), etc. The nucleotide of the anticipated gene can be confirmed using a sequencer or the like, as necessary.

Thereafter, the thus obtained genes are ligated to one another in a predetermined order. First, each of the aforementioned genes is cleaved with known restriction enzymes. The thus cleaved DNA fragment of a heparin-binding EGF gene is inserted into a known vector according to a known method. Thereafter, the DNA of a promoter is cleaved with restriction enzymes and the like, and the cleaved portion is then inserted into a site located upstream of the previously inserted gene, at which the aforementioned promoter is able to function. For the binding of DNA, DNA ligase can be used. Examples of a vector include: plasmids derived from *Escherichia coli,* such as pCR4, pCR2.1, pBluescript, pBR322, pBR325, pUC12, or pUC13; plasmids derived from *Bacillus subtilis,* such as pUB 110, pTP5, or pC 194; plasmids derived from yeast, such as pSH19 or pSH15; bacteriophages such as λ phage; and animal viruses such as retrovirus, vaccinia virus, or baculovirus. Other than these viruses, pA1-11, pXT1, pRc/CMV, pRc/RSV, pcDNAI/Neo, etc. can be used. In the present invention, pBluescript, pAT153, etc. are preferably used.

### (4) TRECK method

TRECK method (toxin receptor-mediated cell knockout method) is able to produce a transgenic animal, in which a diphtheria toxin receptor gene is specifically expressed in a target cell population, and it is able to give damage to the above target cell population by administration of diphtheria toxin at any given time in a quantitative manner. Thus, this method is a novel creative method for clarifying the roll of a cell line in an individual animal. The principle of such TRECK method will be described below.

The amino acid sequence that constitutes HB-EGF in the case of Primates such as a human or a monkey slightly differs from that in the case of rodents such as a mouse or a rat. Binding affinity for diphtheria toxin obtained when such diphtheria toxin is incorporated into a cell by receptor-dependent endocytosis also differs between Primates such as a human or a monkey and rodents such as a mouse or a rat. For example, a mouse cell shows resistance to diphtheria toxin. This is because, since mouse HB-EGF has affinity for diphtheria toxin that is significantly lower than the case of a human receptor, its function as a diphtheria toxin receptor becomes extremely weak, and thus diphtheria toxin cannot bind to the above receptor and thereby cannot come into a cell. However, if human-derived HB-EGF (hHB-EGF) acting as a diphtheria toxin receptor is allowed to express in a mouse cell, because of the human-derived HB-EGF introduced into the mouse cell, diphtheria toxin can be incorporated into the cell just as with the case of a human. As a result, even such a mouse cell becomes sensitive to diphtheria toxin. Utilizing this property, a mouse wherein hHB-EGF has been allowed to express in a target cell population is produced, and diphtheria toxin is then administered to the mouse when damage is intended to be given thereto. Thus, only the target cells can be eliminated, or temporal damage can be given to the cells.

Specifically, diphtheria toxin is a protein having a molecular weight of 58,000, and it consists of fragment A and fragment B. Once such diphtheria toxin is incorporated into a cell, fragment A causes ADP-ribosylation of a peptide chain elongation factor 2 (EF-2) acting as a target molecule, and thus it inactivates EF-2. As a result, protein synthesis is inhibited, resulting in cell death. A mouse cell shows resistance to diphtheria toxin. This is because mouse HB-EGF does not function as a diphtheria toxin receptor, and thus such diphtheria toxin cannot come into a mouse cell. Thus, if human-derived HB-EGF (hHB-EGF) acting as a diphtheria toxin receptor is allowed to express in a mouse cell, even such a mouse becomes sensitive to diphtheria toxin. The TRECK method utilizes this property. A mouse wherein hHB-EGF has been allowed to express in a target cell population is produced, and diphtheria toxin is then administered to the mouse when damage is intended to be given to the cells. Thus, only the target cells can be eliminated, or temporal damage can be given thereto.

Specific experimental procedures of the TRECK method are as follows. That is to say, a plasmid having the promoter region of a gene that is specifically expressed in only a target cell population is produced. Such a plasmid may comprise an enhancer region, as necessary. At that time, such a plasmid is prepared by CsCl equilibrium density gradient centrifugation or the like, and a gene portion to be introduced is then cleaved with restriction enzymes. Thereafter, the cleaved portion is separated by agarose gel electrophoresis, and is then purified. The gene to be introduced is used in production of a transgenic animal.

Diphtheria toxin may be administered to the produced transgenic animal via any one of intravenous, intraperitoneal, subcutaneous, or intramuscular administration. The dosage of diphtheria toxin differs depending on the type of an animal used. In the case of a mouse for example, even if diphtheria toxin is administered at a dosage of 50 µg/kg, no abnormity is observed. Thus, the dosage of diphtheria toxin is generally between approximately 50 ng/kg and 5 µg/kg, which corresponds to 1/1000 to 1/10 of 50 µg/kg. Further, with regard to the timing of administration, diphtheria toxin can be administered when the introduced gene is intended to be disrupted.

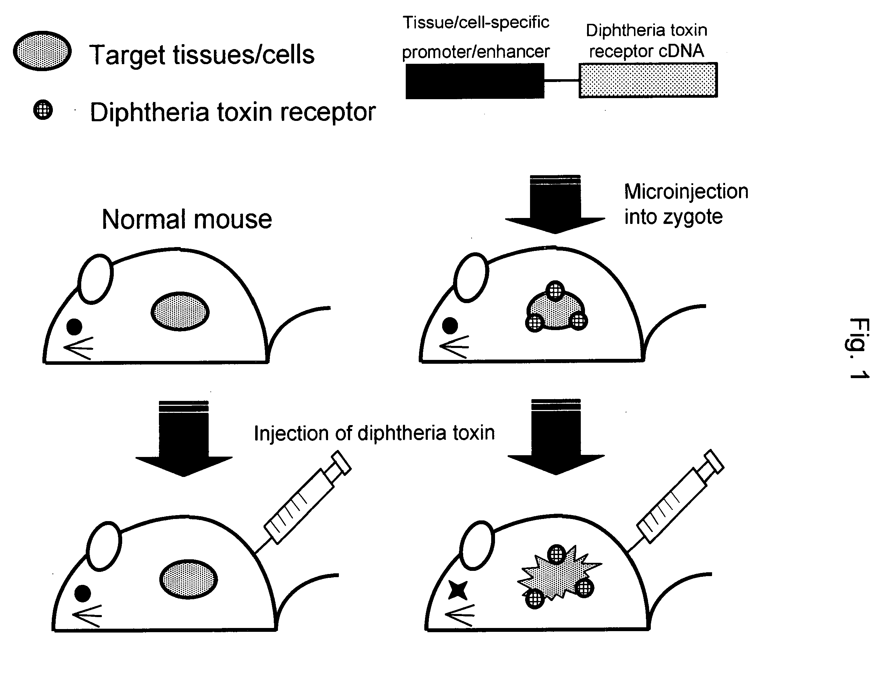

Figure 1 is a schematic view showing the case of disintegrating target tissues or cells by the TRECK method of the present invention. In Figure 1, in order to develop diabetes, an insulin promoter may be used as a tissue/cell-specific promoter/enhancer.

### (5) Diabetes

Diabetes involves a state in which the level of glucose in blood excessively increases (hyperglycemia) due to insufficient insulin action, namely, shortage of insulin supply, and a decrease in sensitivity in an insulin target organ, and as a result, glucose floods into urine (glucosuria). Diabetes is classified into the following types: (i) type 1 diabetes, wherein insulin cannot be secreted as a result of disruption of pancreatic β cells by a certain cause during childhood, and wherein the disease relatively drastically develops as hyperglycemia; (ii) insulin-nondependent type II diabetes, which develops as a result of a low level of insulin secretion or the action of insulin to decrease blood glucose that has become weak, and with which several factors such as dietary life, insufficient exercise, and stress, as well as a hereditary factor, are highly associated; and (iii) pregnancy diabetes, which is triggered by pregnancy. Of these, the transgenic nonhuman animal of the present invention develops type I diabetes.

### 2. Transgenic nonhuman animal

The transgenic nonhuman animal of the present invention is characterized in that it develops diabetes as a result of administration of diphtheria toxin. A method of producing the transgenic nonhuman animal of the present invention and the phenotype thereof will be described below.

### (1) Method of producing transgenic nonhuman animal

The transgenic nonhuman animal of the present invention can be obtained by introducing the aforementioned recombinant DNA into the genome of a nonhuman animal using genetic recombination technology. The type of the animal used in the present invention is not particularly limited. Examples of such an animal include experimental animals or domestic animals, such as a mouse, a rat, a guinea pig, a hamster, a dog, a cat, a swine, a sheep, a goat, a bovine, or a horse.

In the present invention, a mouse is preferably used because of its handlability and reproductivity. Hereafter, a mouse is given as an example of such a transgenic nonhuman animal.

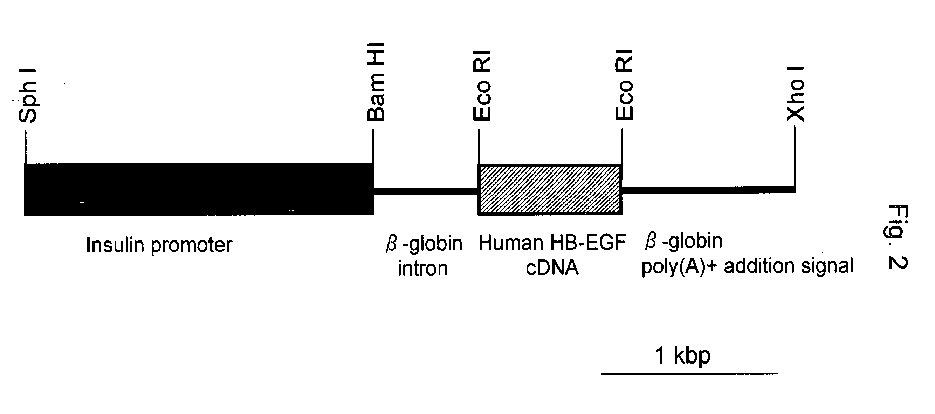

A transgenic nonhuman animal can be produced by a standard method using a mouse, such as a microinjection method using a zygote or an introduction method using ES cells. In the microinjection method, a cloning vector containing a gene to be introduced, which is used in microinjection for production of a transgenic animal, such as an expression cassette as shown in Figure 2 of the present invention, is produced. Subsequently, the expression cassette is cut out of the above expression vector by cleavage with restriction enzymes or the like. Thereafter, a purified DNA fragment is injected into the pronucleus of a fertilized mouse egg cell according to standard methods (for example, Hogan, B. et al., (1994) Manipulating the Mouse Embryo 2nd edn., Cold Spring Harbor Laboratory Press, Cold Spring Harbor, New York).

In order to identify a transgenic mouse, genomic DNA is prepared from ear-punched pieces or tail tissues. That is to say, such ear-punched pieces or pieces of tail tissues are placed in a mixture of a PCR buffer, a nonionic detergent, and proteinase K (50 mM KCl, 10 mM Tris-HCl, (pH8), 1.5 mM MgCl₂, 0.1% gelatin, 0.45% NP-40, 0.45% Tween-20, and 100 µg/ml proteinase K). The obtained mixture is incubated at 55°C overnight. After completion of the incubation, in order to deactivate proteinase K, the reaction product is incubated at 95°C for 15 minutes. The thus obtained solution is used as a DNA sample. In order to confirm the introduced gene, for example, primers (having the following nucleotide sequences, for example) are used to amplify a portion of the introduced recombinant gene of the present invention:
5'- CAACTACATCCTGGTCATCATC -3' (SEQ ID NO: 4); and
5'- CAGACAGATGACAGCACCACAG -3' (SEQ ID NO: 5).

Using the introduced recombinant gene as a template, a PCR reaction is carried out for amplification. For such a PCR reaction, TaKaRa Ex-Taq polymerase (Takara Bio Inc.) and a reaction solution included therewith are used. As a PCR reaction, after incubation at 96°C for 2 minutes, a cycle consisting of 96°C-30 seconds, 55°C-30 seconds, and 72°C-1 minute is repeated 35 times, followed by a reaction at 72°C for 7 minutes, so as to amplify a DNA fragment of interest.

In the introduction method using ES cells, the obtained recombinant DNA is introduced into ES cells. Such ES cells are derived from the inner cell mass of a zygote at the blastocyst stage. These cells can be cultured, while they remain in an undifferentiated state *in vitro.* As such ES cells, both the previously established cell line and a newly established cell line can be used. Examples of such a cell line used herein include a TT2 cell line, an AB-1 cell line, a J1 cell line, and an R1 cell line. The ES cell line used herein can be appropriately selected from among the above cell lines, depending on the purpose of an experiment or a method applied. For introduction of a gene into ES cells, methods such as calcium phosphate coprecipitation, electroporation, lipofection, retrovirus infection, agglutination, microinjection, or particle gun can be adopted. Among others, electroporation is preferable because it enables easy treatment of a large number of cells.

When recombinant DNA is introduced into ES cells, homologous recombination occurs between a specific gene in a portion that is homologous to the recombinant DNA in the genome of the ES cells and the recombinant DNA, so that the gene of the recombinant DNA can be introduced into the ES cells.

ES cells, into which a gene to be introduced has been incorporated, can be tested by the screening of chromosomal DNA separated and extracted from colonies obtained by culturing a single cell on feeder cells according to Southern hybridization or the PCR method. Otherwise, a vector that contains a drug resistance gene or a reporter gene may also be used for selection. Examples of such a drug resistance gene include a neomycin phosphotransferase II (nptII) gene and a hygromycin phosphotransferase (hpt) gene. Examples of such a reporter gene include a β-galactosidase (lacZ) gene and a chloramphenicol acetyl transferase (cat) gene.

ES cells, in which incorporation of a gene to be introduced has been confirmed, are returned to the embryo derived from a mouse of the same species, so that the ES cells can be incorporated into a cell mass of the host embryo, thereby forming a chimeric embryo. The chimeric embryo is then transplanted to a host parent, so that it is allowed to develop and grow therein, thereby obtaining a chimeric transgenic mouse. Thereafter, a chimeric mouse is selected from among offsprings born from the host parent. (In the case of a mouse, offsprings are born after approximately 17 days.) In general, an animal having a high contribution ratio of chimeric animals is highly likely to be a germ line. A possible chimeric mouse is mated with a normal mouse, so as to confirm that it is a germ-line chimeric mouse. Thereafter, the thus obtained germ-line chimeric mouse is mated with a normal female mouse to obtain the F1 generation, so as to establish a mutant animal line.

The thus obtained chimeric transgenic mouse is a heterozygote, which has the introduced gene on only one chromosome of each homologous pair. In order to obtain a homozygote, which has recombinant DNA on the both chromosomes of a homologous pair, two heterozygotes (a brother and a sister) having the introduced DNA on only one chromosome of a homologous pair, which are selected from among F1 animals, may be mated. Introduction of the recombinant DNA can be confirmed by PCR.

The transgenic mouse is subjected to natural crossing or external fertilization for reproduction, so as to produce the transgenic mouse of the present invention. As stated above, the transgenic animal (e.g. transgenic mouse) of the present invention is able to pass its genotype to the progenies thereof via subculture (natural crossing or external fertilization). Accordingly, the transgenic animal of the present invention includes the progenies thereof

### (2) Severe combined immunodeficiency (SCID) animal

The term "severe combined immunodeficiency (hereinafter referred to as SCID at times) animal" is used to mean a spontaneously mutated animal, which is obtained by mutation of subunit P350 of DNA-dependent protein kinase for binding DNA portions that have been fragmented at the stage of rearrangement by genetic recombination, and which has an autosomal recessive mode of inheritance. This animal has been discovered for the first time in C.B-17 mice line by Dr. Melvin Bosma et al. in 1980. Since this mouse does not have T cells and B cells, it exhibits severe immunodeficiency. This mouse shows a low level of rejection reaction to transplantation of heterologous cells or tissues, and thus it is possible to transplant even normal human hematopoietic cells to the mouse. Accordingly, such an SCID animal is not only useful as a animal model of various types of diseases, but also it can be applied to studies regarding phylaxis that utilize immunodeficiency and also to studies using transplanted human cells or tumors. Examples of such an SCID animal include a mouse, a rat, and a dog. As such SCID animals, only SCID mice are commercially available at present, and such SCID mice can be purchased from CLEA Japan Inc., an experimental animal distributor.

Since such an SCID animal is in an immunologically deficient state, human organs and the like can be transplanted thereto.

Previously, there have been the following reports. (i) Gerling et al. have reported that streptozocin is administered to an SCID mouse to cause it to develop diabetes (Diabetes, 43: 433-440, 1994). (ii) Also, it has been reported that the pancreas, bone marrow, or the like of a human or another animal is transplanted to such an SCID mouse, so as to screen and identify the stem cells or precursor cells of pancreatic β cells (e.g. Proc. Natl. Acad. Sci. U.S.A. 2003, 100: 7253-7258. 2003).

Hence, the present inventor has made an attempt to cause such an SCID mouse to develop diabetes, and to transplant the stem cells or precursor cells of pancreatic β cells from various animal species including a human as a typical example to the above SCID mouse. The inventor has considered that a highly reproducible and technically easy system can be established by applying the TRECK method to the aforementioned transplantation method.

Thus, if the recombinant DNA as shown in Figure 2 can be introduced into the genome of the aforementioned SCID animal so as to cause the above animal to develop diabetes, the stem cells or precursor cells of pancreatic β cells from various animal species including a human as a typical example can be transplanted to the above animal. Accordingly, the aforementioned animal is preferable as a diabetes animal model and the like.

### (3) Diabetes animal model

The transgenic nonhuman animal of the present invention develops diabetes as a result of administration of diphtheria toxin thereto. Specifically, diphtheria toxin used to administer to the transgenic nonhuman animal of the present invention is obtained by concentrating the culture supernatant filtrate of *Corynebacterium diphtheriae* (*C. diphtheriae* PW8) via ultrafiltration, precipitation by ammonium sulfate, and purifying the precipitate by DEAE-sepharose column chromatography, followed by appropriate dilution. Such diphtheria toxin can also be obtained from Sigma Aldrich (#D0564).

In general, even if diphtheria toxin is administered at a dosage of 50 µg/kg to a mouse, no abnormality is observed. Thus, diphtheria can be used up to and including the aforementioned dosage. However, it may be better that diphtheria is generally administered at a dosage between approximately 50 ng/kg and 5 µg/kg, which is an amount that is 1/1000 to 1/10 of the aforementioned dosage. A method of administering diphtheria toxin may be any one of intravenous, intraperitoneal, subcutaneous, and intramuscular administrations.

The onset of diabetes can be easily determined by measuring the blood glucose level (glucose level in blood) in the blood that is obtained by puncturing mouse caudal vein with an injection needle, using a blood glucose measurement device, Dexter ZII (Bayer). As such a blood glucose measurement device, other commercially available blood glucose measurement devices that have been widely used for human diabetes patients can also be used. Glucose in urine can be detected using Pretest (Wako Pure Chemical Industries, Ltd.), URIACE (Terumo), or the like.

In order to cause the transgenic nonhuman animal of the present invention to develop diabetes, diphtheria toxin may be intraperitoneally administered at a dosage of 50 ng/kg to 50 µg/kg to the transgenic mouse of the present invention, for example. Specifically, within two days after intraperitoneal administration of diphtheria toxin at a dosage of 50 ng/kg to 50 µg/kg to the transgenic mouse of the present invention, the above transgenic mouse has symptoms specific for diabetes, such as a blood glucose level that has been increased to 200 mg/dl or more and detection of glucose and ketone bodies in urine. The above mouse that has developed diabetes also has symptoms that are frequently observed in human diabetes patients, such as polydipsia, polyuria, and weight reduction. Accordingly, such a mouse that has developed diabetes can be used as a diabetes model mouse.

### 3. Method of screening therapeutic agent for diabetes

In the present invention, a candidate substance (a test substance) for agents used for the treatment of diabetes is brought into contact with (e.g. is administered to) a transgenic animal that has developed diabetes as a result of administration of diphtheria toxin, so as to screen a therapeutic agent for diabetes. For example, a substance used as a candidate for a certain agent is allowed to come into contact with the transgenic nonhuman animal of the present invention, which has developed diabetes as a result of administration of diphtheria toxin, or a portion thereof, and the indicator value (e.g. a blood glucose level, or an insulin level in blood or in other tissues) of a substance having a correlation with a disease as a target is then measured in the above nonhuman animal, with which the above candidate substance has been come into contact, or in a portion thereof. Thereafter, the obtained indicator value is compared with that of a control. Based on the comparative results, it is confirmed whether or not the candidate substance is able to alleviate or eliminate the symptoms of diabetes, so as to screen the candidate substance. Specifically, the blood glucose level of a nonhuman animal, with which a candidate substance has been brought into contact, is measured. When the thus measured blood glucose level is lower than that of a control animal, which has not contact with such a candidate substance, the above candidate substance can be selected as a therapeutic agent used for diabetes.

Moreover, the insulin level of a nonhuman animal, with which a candidate substance has been brought into contact, is measured. When the thus measured insulin level is higher than that of a control animal, which has not contact with such a candidate substance, the above candidate substance can be selected as a therapeutic agent used for diabetes. The types of tissues used in the measurement of insulin are not particularly limited. However, blood is preferable.

The term "a transgenic nonhuman animal or a portion thereof' is used to include both the whole body of a living animal, and specific tissues or organs thereof. In the case of such tissues or organs, those excised from animals are also included. In the present invention, the pancreas is preferable.

Examples of a candidate substance include a peptide, a protein, a nonpeptide compound, a synthetic compound, a fermented product, a cell extract, a cell culture supernatant, a plant extract, a tissue extract and blood plasma of mammal (e.g. a mouse, a rat, a swine, a bovine, a sheep, a monkey, a human, etc.). Such compounds may be either novel compounds or known compounds. These candidate substances may form salts. Examples of such salts of candidate substances include salts with physiologically acceptable acids (e.g. organic acids and inorganic acids, etc.) or with bases (e.g. metal salts, etc.). In particular, physiologically acceptable acid-addition salts are preferable. Examples of such salts include salts with inorganic acids (e.g. hydrochloric acid, phosphoric acid, hydrobromic acid, sulfuric acid, etc.), or salts with organic acids (e.g. acetic acid, formic acid, propionic acid, fumaric acid, maleic acid, succinic acid, tartaric acid, citric acid, malic acid, oxalic acid, benzoic acid, methanesulfonic acid, benzenesulfonic acid, etc.).

Examples of a method of allowing a test animal to come into contact with a candidate substance include oral administration, intravenous injection, topical application, subcutaneous administration, intracutaneous administration, and intraperitoneal administration. Such a method can be appropriately selected, depending on the symptoms of a test animal, the properties of a candidate substance, etc. In addition, the dosage of a candidate substance can be appropriately selected, depending on an administration method, the properties of a candidate substance, etc.

When a certain candidate substance had been administered, for example, if alleviation or elimination of the symptoms of diabetes was confirmed, the used candidate substance could be selected as a therapeutic agent for diabetes.

### 4. Cell transplantation

The present invention provides a method of producing an animal having pancreatic cells derived from another animal, which is characterized in that it comprises transplantation of cells derived from another animal to the aforementioned diabetes animal model.

The term "another animal" is used to mean either an animal of species different from an animal used in production of a diabetes animal model, or an allogenic animal. Examples of the "animal of different species" include a mouse, a monkey, a bovine, a swine, a sheep, a goat, a rabbit, a dog, a cat, a guinea pig, a hamster, a rat, and a horse, as well as a human, but examples are not limited thereto. The "allogenic animal" is used to mean an animal of the same animal species, but of a different strain. For example, if the mouse used in production of a animal model were C3H, a mouse used in transplantation could be C57BL/6.

Examples of cells used in transplantation include pancreatic cells (including α cells and β cells) and undifferentiated cells such as stem cells. Hematopoietic stem cells (in particular, bone marrow cells and cord blood cells) are preferable. When stem cells are transplanted to a recipient, the cells derived from a donor are regenerated in the destroyed pancreatic cells or tissues of the recipient, and the regenerated cells survive therein, so that the recipient is able to restore a normal pancreatic function. The present invention also provides the thus produced transgenic nonhuman animal.

As undifferentiated cells used herein, the ES cells of mice (Nature 292: 154-156, 1981), rats (Dev. Biol. 163(1): 288-292, 1994), monkeys (Proc. Natl. Acad. Sci. U.S.A. 92(17): 7844-7848, 1995), and rabbits (Mol. Reprod. Dev. 45(4): 439-443, 1996) have been established. In addition, with regard to swines, EG (embryonic germ) cells have been established (Biol. Reprod 57(5): 1089-1095, 1997). However, examples of undifferentiated cells are not limited thereto. Whether or not such a cell maintains an undifferentiated state can be confirmed using various types of known markers, which have been established in mammals (e.g. a mouse or a bovine), for example.

Hematopoietic stem cells include bone marrow-derived cells, cord blood-derived cells, and peripheral blood hematopoietic stem cells. The hematopoietic stem cells used in the present invention can be collected from bone marrow, cord blood, or peripheral blood, and can be then purified. As a way to obtain the hematopoietic stem cells from these tissues, a method using various surface antigens existing on a cell surface has been known. As a representative method, the method developed by Osawa et al. has been known (Osawa M, Nakamura K, Nishi N, Takahasi N, Tokuomoto Y, Inoue H, Nakauchi H, In vivo self-renewal of c-Kit+Sca-1+Lin(Low/-) hemopoietic stem cells. J. Immunol. 156: 3207-3214, 1996). In this method, nucleated cells are separated from bone marrow, cord blood, or peripheral blood, and antibodies reacting with surface antigens are allowed to act on the separated cells, followed by separation with a cell separator. The thus obtained cells can be directly cultured. It is to be noted that the hematopoietic stem cells used in the present invention may be undifferentiated.

The number of cells to be transplanted is not limited. When a recipient animal is a mouse, the number of donor cells is for example 10⁴ to 10⁹, and preferably 10⁶ to 10⁸.

Methods of transplanting the aforementioned cells to an animal have been well known, and persons skilled in the art can carry out such transplantation according to such known methods. For example, a method of culturing bone marrow cells and then administering the cells into vein can be adopted.

After completion of the transplantation, the transplanted cell or the transgenic animal of the present invention is observed, and it is evaluated regarding the following items: whether or not the transplanted stem cells have differentiated into desired cells; whether or not the stem cells have been fixed to the tissues, to which they have been transplanted; whether or not the stem cells have become cancerous; whether or not transplantation has affected the health condition of a clone animal; whether or not the transplanted cells have exhibited therapeutic effects on the developed diabetes; the site in the body to which the transplanted cells have moved; etc. For example, the above items are evaluated by observing the transgenic animal of the present invention with the naked eye, by observing the transplanted cells or the peripheral tissues with a microscope, or by detecting the expression of a marker (e.g. a diabetic condition marker gene, a gene acting as an indicator of differentiation into specific cells), etc. In addition, it can be confirmed that the transplanted cells in the animal produced by the aforementioned method are derived not from a recipient but from a donor by detecting the insulin of the donor animal species in blood. For example, mass spectrometry may be carried out to confirm the presence of a molecule that corresponds to the molecular weight of the insulin of the donor animal species. The molecular weight of the donor animal species can be easily obtained by acquiring the nucleotide sequence or amino acid sequence of the gene from public database such as National Center for Biotechnology Information (NCBI) or SwissPlot. Otherwise, the presence of such a molecule can also be confirmed by extracting DNA from a recipient, and then carrying out PCR using primers specific for the donor animal species. Further, the presence of such a molecule can also be confirmed by the immunostaining of a tissue section using an antibody that specifically recognizes a protein derived from the donor animal species (e.g. an anti-cytokeratin 18 antibody, an anti-MafA antibody, an anti-Pdx1 antibody, etc.). When cells derived from a mouse or a rat are transplanted, the presence of the donor cells in the recipient can be easily confirmed by administering DT to the aforementioned animal. Specifically, it can be verified by confirming that the blood glucose level is either decreased or not elevated in the animal after DT administration.

In the present invention, if cells derived from an animal of different species, such as human pancreatic β cells, are transplanted to the aforementioned mouse of the present invention (SCID-Ins-TRECK-Tg mouse) whose pancreatic β cells have been damaged by administration of DT, the human pancreatic β cells survive in the living mouse body. Thus, it becomes possible to produce a animal model (e.g. SCID-Hu (human) mouse model, etc.) having pancreatic β cells derived from a human.

As stated above, a animal model, to which pancreatic cells or stem cells have been transplanted, is used to analyze a differentiation process in which cells derived from the donor achieve the function as pancreatic cells. Thereby, the above animal model can also be used to screen and identify pancreatic cells or stem cells.

The present invention will be more specifically described in the following examples. However, these examples are not intended to limit the scope of the present invention.

### [Example 1] Construction of cloning vector comprising expression cassette

Components that constitute a recombinant gene constructed to produce a diabetes model SCID mouse are aligned in the order of a human insulin promoter, a rabbit β-globin intron, human HB-EGF cDNA, and a rabbit β-globin poly(A⁺) signal. Using a plasmid pRcHBEGF (EMBO J. (1994) 13: 2322-2330) as a template, site-directed mutagenesis was performed, so as to substitute the leucine at position 148 of the synthesized HB-EGF protein with serine, and the proline at position 149 with threonine, thereby obtaining human HB-EGF (L148S/P149T) cDNA (SEQ ID NO: 3). This cDNA was inserted into a site downstream of a human insulin promoter (1.9 kb) of a plasmid pIns-1 (J. Exp. Med. (1998) 188: 1445-1451), so as to construct a plasmid pIns-TR2 (Figure 2).

### [Example 2] Production of transgenic mouse

A DNA fragment to be used in production of a transgenic animal was obtained by double cleavage with the SphI and XhoI of the plasmid pIns-TR2 described in Example 1. The fragment was separated from a cloning vector by agarose gel electrophoresis, and it was then purified with QIAEX II (QIAGEN, CA). The purified DNA fragment was injected into the pronucleus of a fertilized mouse (C.B-17/Icr-scid/scid) egg cell according to standard manners (Hogan, B. et al., (1994) Manipulating the Mouse Embryo 2nd edn., Cold Spring Harbor Laboratory Press, Cold Spring Harbor, New York). In order to identify such a transgenic mouse, genomic DNA was prepared from ear-punched pieces. That is to say, such punched pieces were placed in a mixture of a PCR buffer, a nonionic surfactant, and proteinase K (50 mM KCl, 10 mM Tris-HCl, (pH 8), 1.5 mM MgCl₂, 0.1% gelatin, 0.45% NP-40, 0.45% Tween-20, and 100 µg/ml proteinase K). The obtained mixture was incubated at 55°C overnight. After completion of the incubation, in order to deactivate proteinase K, the reaction product was incubated at 95°C for 15 minutes. The thus obtained solution was used as a DNA sample. In order to confirm the sequence of introduced gene, primers having the following nucleotide sequences were used:
Forward: 5'-CAACTACATCCTGGTCATCATC-3' (SEQ ID NO: 4); and
Reverse: 5'-CAGACAGATGACAGCACCACAG-3' (SEQ ID NO: 5).
Using the introduced recombinant gene as a template, a PCR reaction was carried out for amplification.

For such a PCR reaction, TaKaRa Ex-Taq polymerase (Takara Bio Inc.) and a reaction solution included therewith were used. As a PCR reaction, after completion of a reaction at 96°C for 2 minutes, a cycle consisting of 96°C-30 seconds, 55°C-30 seconds, and 72°C-1 minute was repeated 35 times, followed by a reaction at 72°C for 7 minutes, so as to amplify a DNA fragment (0.7 kb) of interest. After completion of the PCR reaction, agarose gel electrophoresis was carried out to determine whether the introduced gene was present or not.

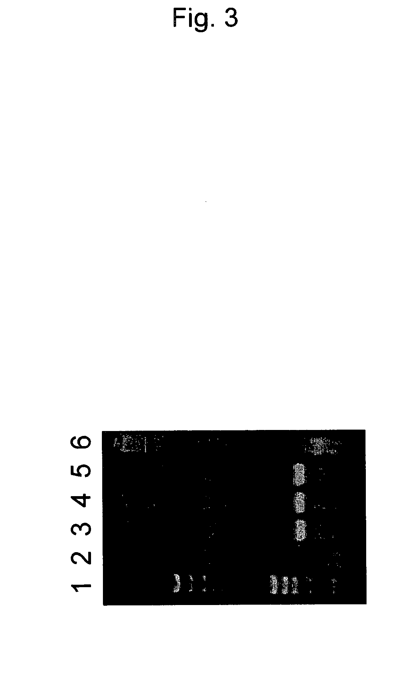

The results are shown in Figure 3 (Lane 1: a DNA size marker (a λDNA HindIII digest + φX174 HaeIII digest mixture); Lane 2: C.B-17/Icr-scid/scid genomic DNA; Lane 3: the introduced gene and C.B-17/Icr-scid/scid genomic DNA; Lanes 4 to 6: sample DNAs). The figure shows that the above gene has been introduced into the 4^{th} and 5^{th} mouse genomes.

### [Example 3] Administration of diphtheria toxin

### (1) Measurement of blood glucose level (glucose level in blood)

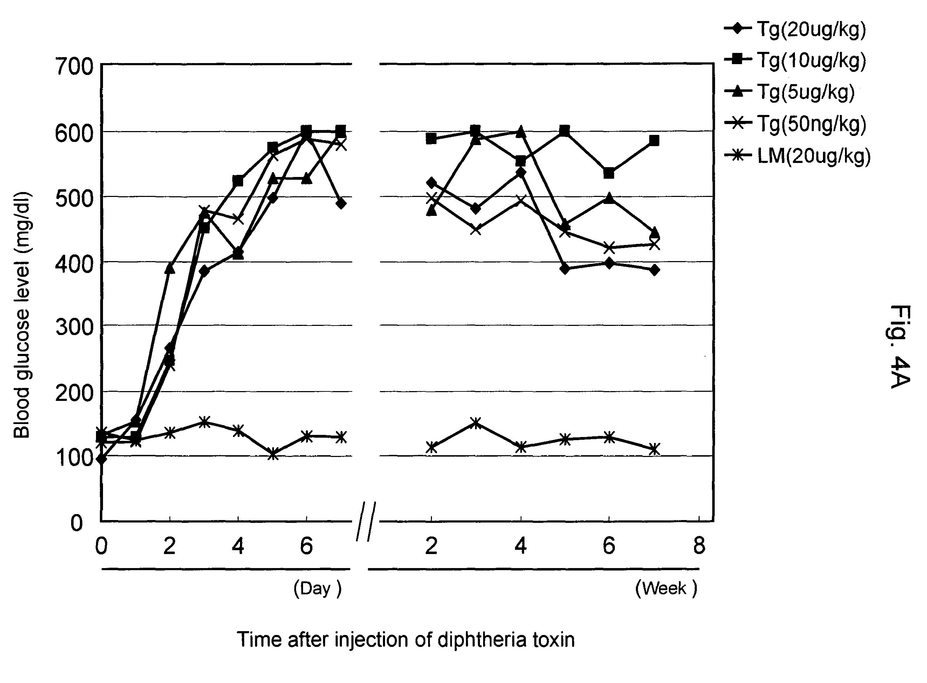

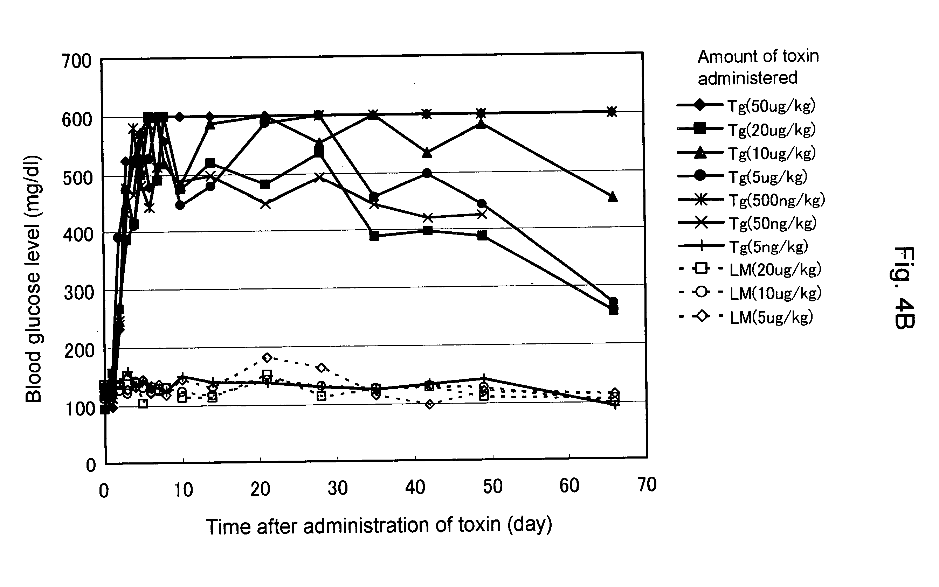

Various concentrations of diphtheria toxins (dissolved in PBS) were intraperitoneally administered to the produced transgenic mice (SCID-Ins-TRECK-Tg mice). Changes in the blood glucose levels were measured using Dexter ZII (Bayer). The results are shown in Figure 4A. In all cases, two days after administration of diphtheria toxin at a dosage of 50 ng/kg to 20 µg/kg, the blood glucose level of such a transgenic mouse was increased to 200 mg/dl or more, and then, from four days after such administration, the blood glucose level was maintained at a high value of 400 mg/dl or more for 50 days or more. In the case of a non-transgenic mouse born of the same mother, no changes were found in the blood glucose levels due to administration of the toxin.

In addition, diphtheria toxin was intraperitoneally administered to the transgenic mice in the same manner as described above with the exception that the amount of diphtheria toxin was reduced (5 ng/kg to 50 µg/kg).

As a result, a hyperglycemic state was sustained for 2 months or more by a single DT administration. These results demonstrated that the minimum DT amount necessary for inducing hyperglycemia is 50 ng/kg (Figure 4B).

### (2) Decrease in blood glucose level by administration of insulin

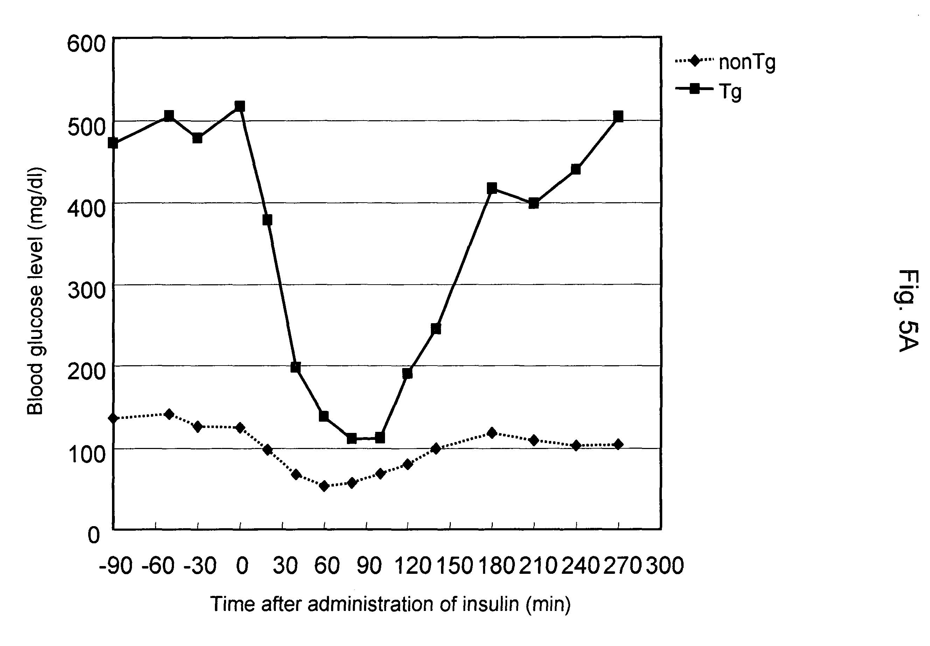

In order to examine whether elevation of the blood glucose level of a transgenic mouse as a result of administration of diphtheria toxin is insulin-dependent or not, a rapid-acting insulin analogue (NovoRapid injection; NovoNordisk Pharma) was administered to a mouse whose blood glucose level had been increased by administration of 1 µg/kg DT. The blood glucose level (500 mg/dl or higher) of the transgenic mouse, which had developed diabetes, was rapidly decreased to a normal value by administration of 100 mU insulin (Figure 5A).

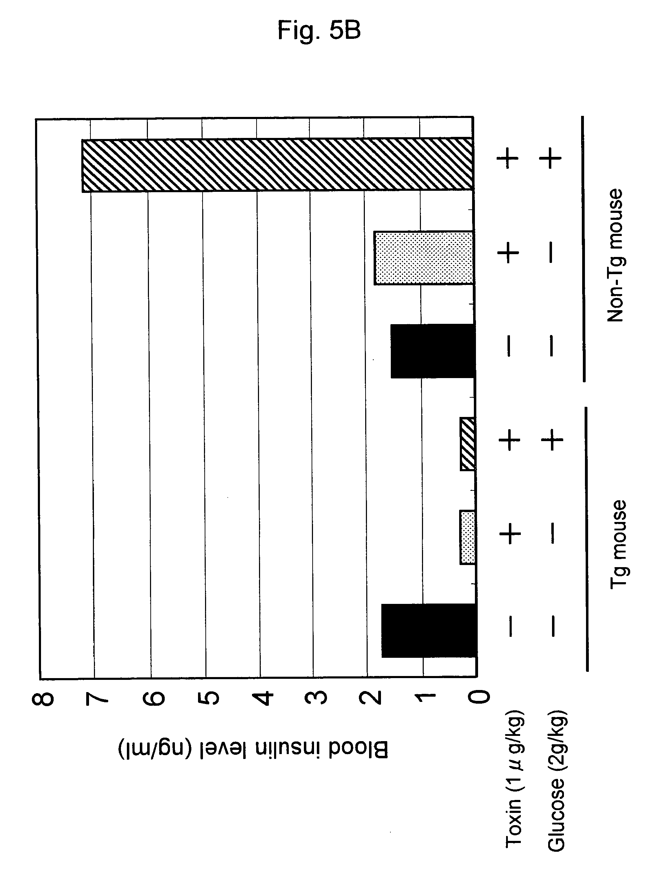

Moreover, the blood insulin level of an SCID-Ins-TRECK-Tg mouse was compared with that of a non-Tg mouse. When DT had not yet been administered, there had been no difference between them. However, on the 5^{th} days after administration of DT, the blood insulin level of the SCID-Ins-TRECK-Tg mouse was significantly decreased, whereas that of the non-Tg mouse was not changed. When 2 g/kg glucose was orally administered to the two types of mice, and the blood insulin level was then measured 10 minutes after the administration, the blood insulin level was rapidly increased in the non-Tg mouse. However, the SCID-Ins-TRECK-Tg mouse was not able to secrete insulin, and thus the blood insulin level thereof was not changed (Figure 5B) in it.

Subsequently, 1 µg/kg DT was administered to the SCID-Ins-TRECK-Tg mice. Seven days after the administration, a rapid-acting insulin analogue (NovoRapid injection) was intraperitoneally administered to the thus produced hyperglycemia-induced mice. In the case of a mouse having a blood glucose level of 500 mg/dl, the blood glucose level was normalized by administration of 100 mU insulin. On the other hand, in the case of a mouse having severe hyperglycemia (blood glucose level >600 mg/dl), no changes were found in the blood glucose level by administration of 100 mU insulin. The blood glucose level of such mouse was decreased to a normal level by administration of 1 U of insulin. Accordingly, it was demonstrated that the hyperglycemia of the SCID-Ins-TRECK-Tg mouse is insulin-dependent (Figure 5C).

### (3) Histological analysis of transgenic mouse pancreas

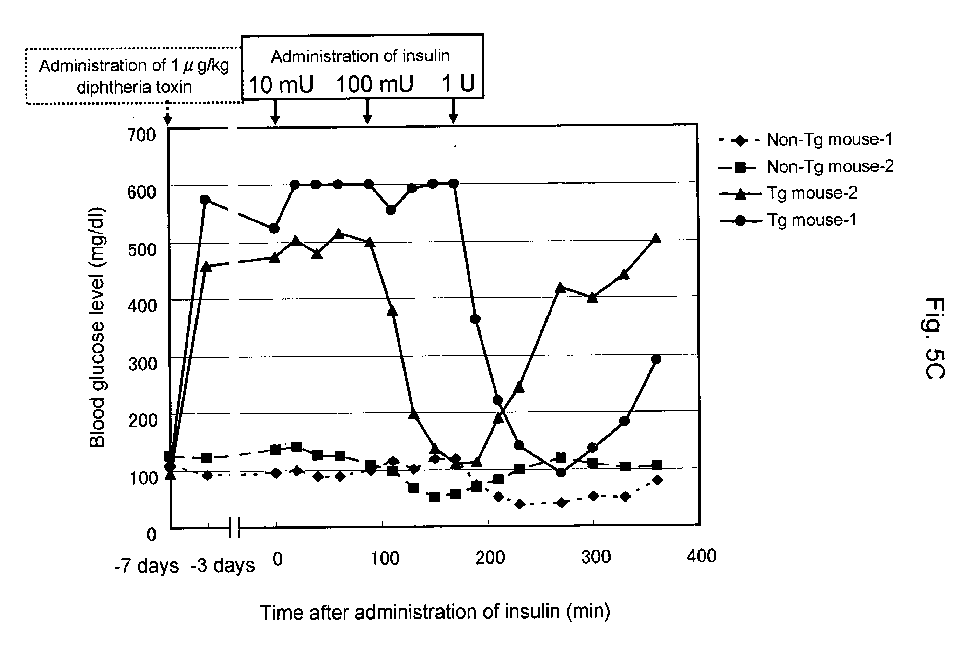

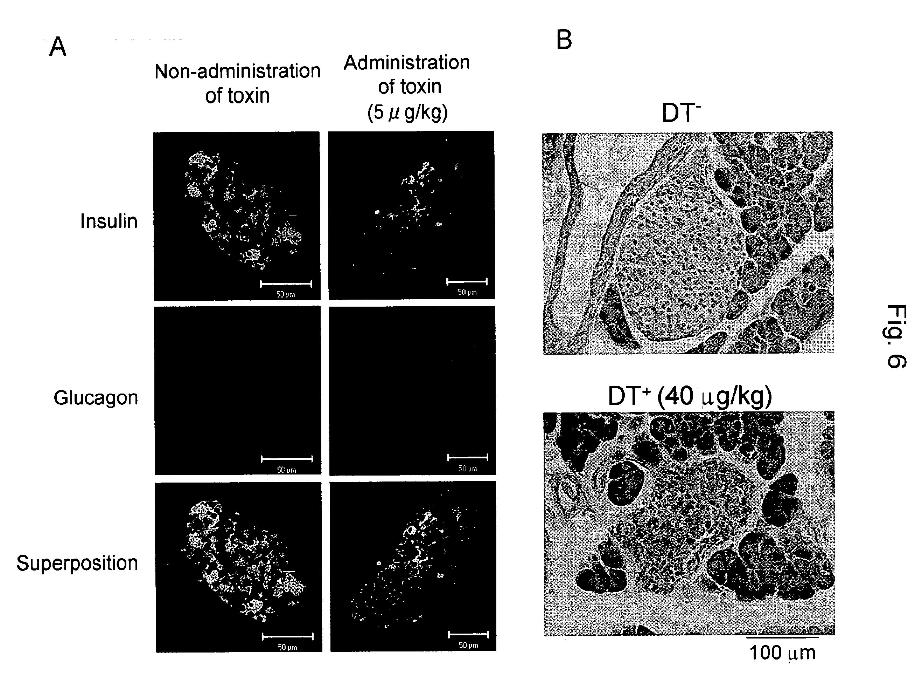

Mice were subjected to euthanasia by cervical dislocation, at the timing before administration of diphtheria toxin and at the timing 7 days after administration of 5 µg/kg diphtheria toxin. Pancreas was excised from each mouse, and it was then immobilized with 10% neutral buffered formalin fixative overnight. Thereafter, the pancreas was embedded in paraffin according to a common method, and it was then processed into a section with a thickness of 5 µm using a microtome. Guinea Pig Anti-Swine Insulin (DAKO) and Rabbit Anti-Human Glucagon (DAKO) were used as primary antibodies. Alexa Fluor 488 goat anti-guinea pig IgG (H+L) and Alexa Fluor 594 goat anti-rabbit IgG (H+L) were used as secondary antibodies. The above section was double-stained with such antibodies, and it was then observed under a laser scanning confocal microscope. Before administration of the toxin, approximately 70% of the cells existing in the islets of Langerhans in the pancreas of the transgenic mouse were β cells stained with anti-insulin antibodies, like normal mouse. After administration of the toxin, however, such insulin positive cells were significantly decreased, and the number of α cells stained with anti-glucagon antibodies was increased (Figure 6, Panel A).

Mice were subjected to euthanasia by cervical dislocation, at the timing before administration of diphtheria toxin and at the timing 7 days after administration of 40 µg/kg diphtheria toxin. Pancreas was excised from each mouse, and it was then immobilized with 10% neutral buffered formalin fixative overnight. Thereafter, the pancreas was embedded in paraffin according to a common method, and it was then processed into a section with a thickness of 5 µm using a microtome. Thereafter, the section was subjected to HE staining, and it was then observed under a microscope. As a result, it was found that the islets of Langerhans of the Tg mouse before administration of DT was normal, but that the cells in the islets of Langerhans of the Tg mouse after administration of DT were damaged (Figure 6, Panel B).

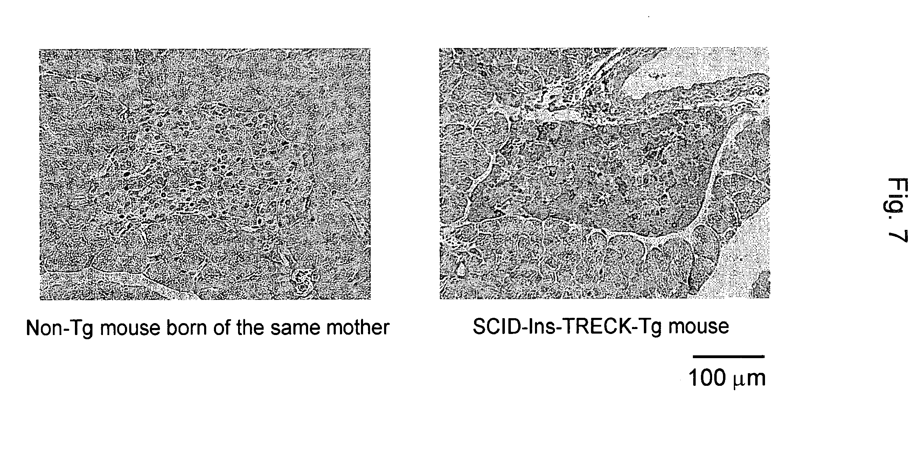

Subsequently, the pancreatic section of a diabetes model mouse (SCID-Ins-TRECK-Tg mouse) and that of an SCID mouse born of the same mother were immunostained with anti-hHB-EGF antibodies. As a result, cells capable of being stained with hHB-EGF antibodies were found only in the islets of Langerhans of the SCID-Ins-TRECK-Tg mouse (Figure 7).

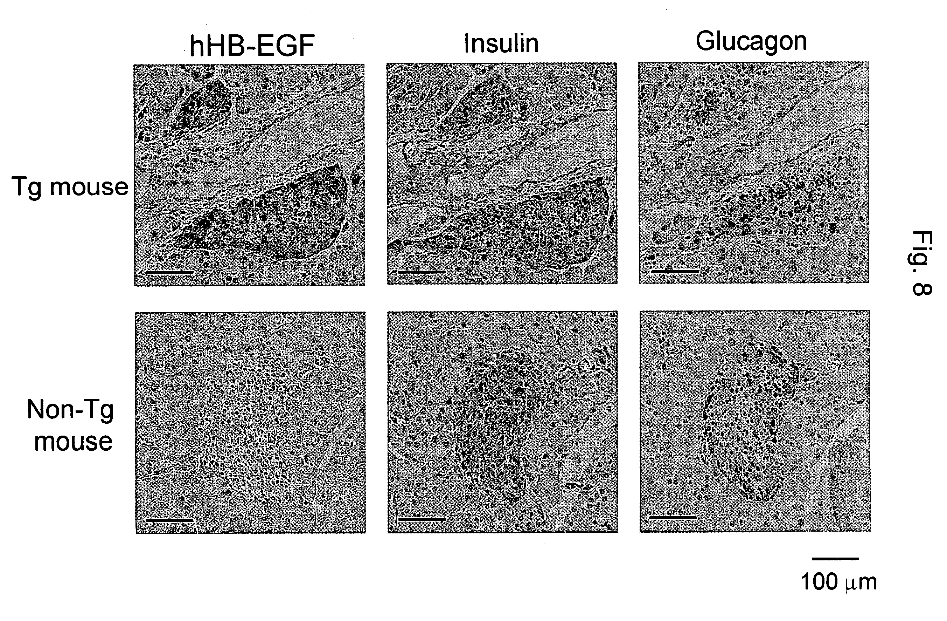

Further, a Tg mouse pancreatic section and a non-Tg-SCID mouse pancreatic section were immunostained with each of an anti-hHB-EGF antibody, an anti-insulin antibody, and an anti-glucagon antibody. As a result, β cells capable of being stained with the insulin antibody were stained with the hHB-EGF antibody. Expression of hHB-EGF was not observed in the β cells of the non-Tg-SCID mouse (Figure 8).

### [Example 4] Transplantation of hematopoietic cells to diabetes model mouse

### (1) Transplantation of bone marrow cells

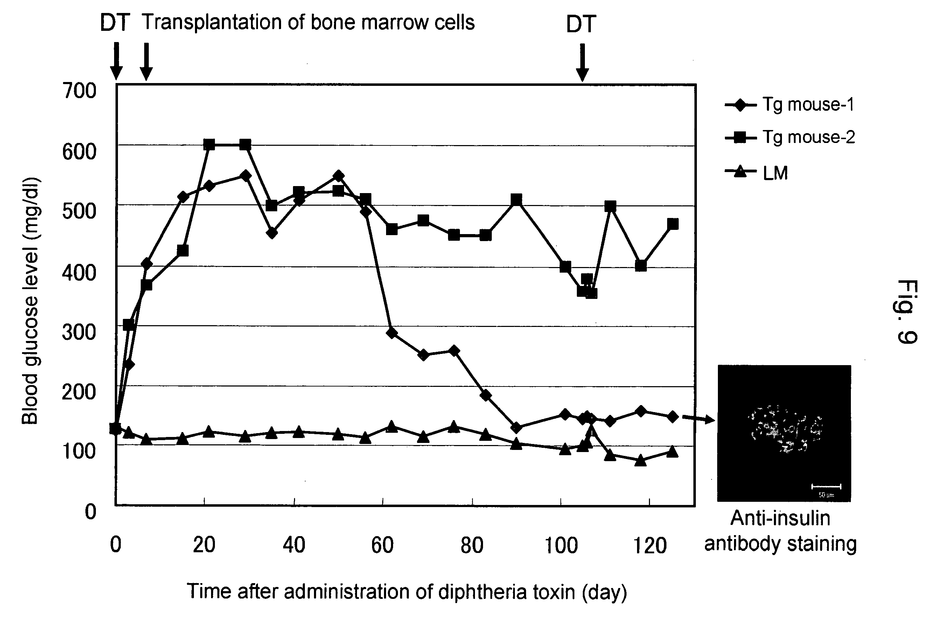

The bone marrow cells (2 x 10⁷ cells) of a C57BL/6 mouse, from which T cells had been eliminated, were transfused to a hyperglycemia-induced mouse via its caudal vein on the 7^{th} day after administration of DT. Figure 9 shows that the blood glucose level of Tg mouse-2 (■) was significantly decreased 9 weeks (54 days) after the transplantation, and that the above blood glucose level became almost a normal value 12 weeks (82 days) after the transplantation. Accordingly, it was demonstrated that hyperglycemia was improved by transplantation of the C57BL/6 mouse bone marrow cells.

From the viewpoint of histochemistry as well, β cells were regenerated in the pancreatic islets of Langerhans of the above hyperglycemia-induced mouse (Figure 9, the photograph). Although DT was administered to this mouse again, the blood glucose level was not increased. Thus, it was demonstrated that the regenerated pancreatic β cells were not derived from the recipient, but were derived from the C57BL/6 mouse as a donor.

### (2) Transplantation of human cord blood-derived undifferentiated cells

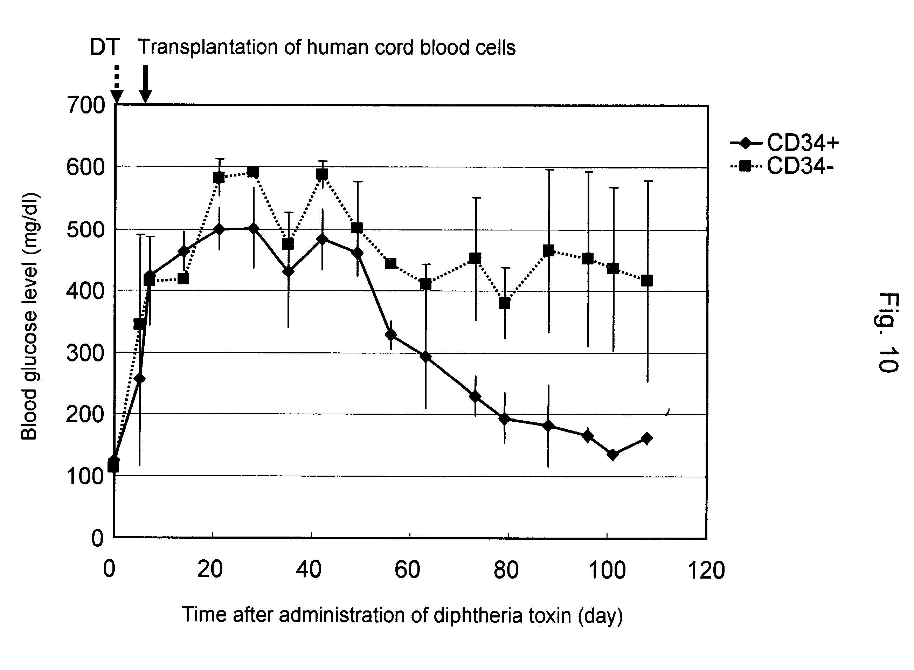

Human cord blood-derived CD34 positive cells or CD34 negative cells were transfused to an SCID-Ins-TRECK-Tg mouse, which had developed hyperglycemia as a result of administration of DT, via its caudal vein on the 7^{th} day after administration of DT. Thereafter, the blood glucose level thereof was measured over time. In the case of a mouse to which the CD34 negative cells had been transfused, the blood glucose level was 300 mg/dl or higher even 100 days after the transfusion. In contrast, in the case of a mouse to which the CD34 positive cells had been transfused, the blood glucose level was decreased from 40 days after the transfusion, and it was 200 mg/dl or lower 70 to 80 days after the transfusion (Figure 10).

Accordingly, it was demonstrated that, as a result of transplantation of human cord blood-derived cells, such human-derived cells survived in the mouse, and that the human-derived cells were then differentiated into pancreatic cells, so that the functions of the pancreatic cells of the mouse could be recovered and hyperglycemia was thereby improved.

### INDUSTRIAL APPLICABILITY

The diabetes animal model of the present invention sufficiently satisfies 3 conditions necessary for a animal model of type I diabetes that utilizes regenerative medicine, namely, (i) it reliably induces diabetes, (ii) it has no abnormality other than diabetes after toxin administration, and (iii) other mammalian cells can be transplanted thereto. Thus, the diabetes animal model of the present invention is highly useful, and it has an extremely favorable effect of being used in investigation of the pathologic conditions of diabetes and the development of a therapeutic agent used for diabetes.

Moreover, when the pancreatic β cells of the diabetes animal model of the present invention have been damaged by administration of DT and thereafter human pancreatic β cells are transplanted to the above animal, the human pancreatic β cells are able to survive in the living body of the mouse. Accordingly, the animal model of the present invention is extremely useful as an SCID-Hu (human) model.
Sequence Listing Free Text

| | |
|---|---|
| SEQ ID NO: 3 | Mutant |
| SEQ ID NO: 4 | Primer |
| SEQ ID NO: 5 | Primer |

## Claims

1. A transgenic nonhuman animal, into which recombinant DNA comprising a gene encoding a diphtheria toxin receptor and an insulin promoter for regulating expression of said gene has been introduced.

2. The transgenic nonhuman animal according to claim 1, which is **characterized in that** it develops diabetes as a result of administration of diphtheria toxin.

3. The transgenic nonhuman animal according to claim 1, wherein the gene encoding a diphtheria toxin receptor is a heparin-binding EGF gene derived from Primates.

4. The transgenic nonhuman animal according to claim 1, wherein the gene encoding a diphtheria toxin receptor is a human heparin-binding EGF gene.

5. The transgenic nonhuman animal according to claim 1, wherein the animal is a severe combined immunodeficiency animal.

6. The transgenic nonhuman animal according to claim 1, wherein the animal is any one selected from the group consisting of a mouse, a rat, a guinea pig, a hamster, a dog, a cat, a goat, a sheep, a swine, a bovine, and a horse.

7. The transgenic nonhuman animal according to claim 1, wherein the animal is a mouse.

8. A method of producing a transgenic nonhuman animal, which is **characterized in that** it comprises introduction of recombinant DNA comprising a gene encoding a diphtheria toxin receptor and an insulin promoter for regulating expression of said gene into a nonhuman animal.

9. A method of producing a diabetes animal model, which is **characterized in that** it comprises administration of diphtheria toxin to the transgenic nonhuman animal according to any one of claims 1 to 7 or a transgenic nonhuman animal produced by the method according to claim 8.

10. A method of causing the transgenic nonhuman animal according to any one of claims 1 to 7 or a transgenic nonhuman animal produced by the method according to claim 8 to develop diabetes, which comprises administration of diphtheria toxin to said transgenic nonhuman animal.

11. A diabetes animal model, which is produced by the method according to claim 9.

12. A method of screening a therapeutic agent used for diabetes, which is **characterized in that** it comprises administration of a candidate substance to the diabetes animal model according to claim 11 or a portion thereof.

13. The method according to claim 12, which comprises: measuring the blood glucose level of a diabetes animal model or a portion thereof, with which a candidate substance has been allowed to come into contact; and then selecting said candidate substance as a therapeutic agent used for diabetes, when the thus measured blood glucose level is lower than the blood glucose level of a control diabetes animal model, with which such a candidate substance has not been allowed to come into contact.

14. The method according to claim 12, which comprises: measuring the insulin level of a diabetes animal model or a portion thereof, with which a candidate substance has been allowed to come into contact; and then selecting said candidate substance as a therapeutic agent used for diabetes, when the thus measured insulin level is higher than the insulin level of a control diabetes animal model, with which such a candidate substance has not been allowed to come into contact.

15. A method of producing a transgenic nonhuman animal having pancreatic cells derived from another animal, which is **characterized in that** it comprises transplantation of stem cells derived from the other animal to the animal according to claim 11.

16. The method according to claim 15, wherein the stem cells are hematopoietic stem cells.

17. The method according to claim 16, wherein the hematopoietic stem cells are bone marrow-derived cells or cord blood-derived cells.

18. A transgenic nonhuman animal having pancreatic cells derived from another animal, which is produced by the method according to claim 15.

19. The transgenic nonhuman animal according to claim 18, wherein the pancreatic cells are derived from the transplanted hematopoietic stem cells of another animal.

20. The transgenic nonhuman animal according to claim 19, wherein the hematopoietic stem cells are bone marrow cells or cord blood cells.
